# EUROPEAN PATENT SPECIFICATION

(11) **EP 3 407 777 B1**
(45) Date of publication and mention of the grant of the patent: **30.12.2020**
(21) Application number: 17744828.9
(22) Date of filing: 25.01.2017
(51) Int. Cl.: A61B 5/00, A61B 8/00, A61B 8/12, A61B 17/00, A61B 17/22, A61B 17/3207, A61B 5/02, A61B 17/32, A61B 90/00

(54) **OCT IMAGING CATHETER WITH LAG CORRECTION**
OCT-BILDGEBUNGSKATHETER MIT LAG-KORREKTUR
CATHÉTER D'IMAGERIE OCT AVEC CORRECTION DE DÉCALAGE

(30) Priority: 25.01.2016 US 201662286918 P
(43) Date of publication of application: 05.12.2018
(73) Proprietor: Avinger, Inc., Redwood City, CA 94063 (US)
(72) Inventor: CHRISTENSEN, Bjarne, B., Redwood City CA 94063 (US)
(74) Representative: Potter Clarkson
(86) International application number: PCT/US2017/014921
(87) International publication number: WO 2017/132247

(56) References cited:
- EP-A1- 2 942 028
- WO-A1-2009/009799
- WO-A1-2012/057940
- WO-A1-2015/120146
- WO-A2-2011/003013
- US-A1- 2003 120 150
- US-A1- 2011 071 401
- US-A1- 2014 343 410
- US-B1- 6 454 717
- US-B2- 7 729 745

## Description

### FIELD

Described herein are image correction systems that can be coupled to imaging catheters. More specifically, imaging correction systems and related methods that correct for rotational distortions experienced by imaging catheters are described.

### BACKGROUND

Atherectomy is a minimally invasive endovascular surgery technique that removes atherosclerosis (plaque buildup in the blood vessels). By removing the majority of plaque mass (debulking), atherectomy creates a larger internal lumen and dramatically increases the compliance of the arterial wall. As a result, stent deployment is greatly enhanced.

Additionally, atherectomy provides several advantages related to the arterial healing response. When circumferential radial forces are applied to the vasculature, as in the case of angioplasty or stenting, the plaque mass is displaced, forcing the vessel wall to stretch dramatically. This stretch injury is a known stimulus for the cellular in-growth that leads to restenosis. By using atherectomy to remove the disease with minimal force applied to the vessel, large gains in lumen size can be created with decreased vessel wall injury and limited elastic recoiling. These effects have been shown to generate better acute results and lower restenosis rates.

Atherectomy devices can be accompanied by image guidance, such as optical coherence tomography (OCT). Having imaging capabilities provide for safer and more targeted treatment. Image guidance is particularly useful in showing the operator where plaque masses are located and to focus debulking where the plaque is visualized, greatly decreasing the risk associated with atherectomy. One additional benefit of having an accompanying visualization system is that the catheter operator does not have to treat the lesions or plaque as being all concentric in nature, when in fact, the majority of the lesions and plaque found are eccentric. This circumferential approximation approach to removing lesions and plaque may not fully remove plaque from one region and potentially cut or stretch healthy vessel. Thus, the ability to visualize the plaque or lesions in the vessel will allow the operator to debulk only in the required areas while leaving healthy tissue untouched.

While having an imaging system coupled to the atherectomy catheter allows for safer procedure than without, on-board imaging can have inaccuracies. For example, placement of the imaging sensor may affect accuracy. That is, imaging elements positioned at or closer to the distal end of the atherectomy catheter can experience greater bending or torque than is experienced at the proximal end. Further, if the imaging element is positioned on or proximate to the cutting element, then when the cutting portion experiences resistance from plaque buildup in the vessel or from the curvature of the vessel itself, deviations can occur in the image. Similarly, a catheter moving through a body cavity or lumen may also experience torque depending on the body lumen and the environment within that body lumen, which can cause distortions in the images.

Additionally, rotational distortions can occur as a result of the cutter turning more slowly than the motor at the proximal end, the cutter vibrating, or the cutter jumping when encountering tissue of different characteristics. These various deviations in the images can be caused by what is called "rotational lag," i.e., a lag between the rotation that the user may or may not have intentionally applied to the catheter and the actual and potentially unintentional rotation of the imaging sensor at the distal end of the catheter. Such rotational lag can be enhanced in some catheters, as the rotational position of the displayed image may be fixed in some catheters according to an index position relative to the catheter motor rather than the distal end of the catheter. Accordingly, there is a need for an imaging atherectomy catheter that accounts or adjusts for rotational lag.

One method previously disclosed for adjusting images distorted by rotational lag was to manually rotate the catheter to compensate for the lag between the rotation applied by the user at the proximal end and the actual rotation of the distal end of the catheter. However, this method does not provide a consistent way of compensating for the rotational lag experienced, as manual adjustment of the catheter will likely differ from user to user as well as in response the uniquely different resistance experienced in different body lumen. Another method previously disclosed for dealing with rotational lag was to separate the images into both a depth versus time image, called a waterfall image, and an azimuthal image that contains angular rotation information. While this method allows the user to see both types of images (showing both azimuthal and time versus depth information), it is not an intuitive way to view the interior of a vessel.

Thus, it would be advantageous to have an atherectomy catheter system with imaging that is able to adjust for rotational lag instantaneously and dynamically. Furthermore, it would also be more cost-effective and accurate to implement a system for correcting rotational lag that utilizes a simple measurement of existing condition to determine if correction is needed. Having a simple measurement that correlates to rotational lag prior to an image being taken would remove the need for having applications and programs to calculate and adjust the image at the output end.

WO 2009/009799 discloses a catheter for in vivo imaging. The catheter comprises a monolithic outer sheath that terminates in a monolithic atraumatic tip having a guidewire lumen, a rotary drive shaft that passes through a central lumen of the outer sheath to impart rotary motion and an optical train positioned within the central lumen.

### SUMMARY OF THE DISCLOSURE

The present invention, the scope of which is defined by the appended claims, provides a catheter system includes a catheter body, an imaging sensor, a drive motor, a current sensor, a display, and a controller. The catheter body includes a drive shaft. The imaging sensor is fixed relative to the distal end of the driveshaft and is configured to rotate therewith. The drive motor is configured to rotate the drive shaft. The current sensor is configured to measure an amount of current drawn by the drive motor as the drive shaft is rotated. The display is configured to display one or more images obtained by the imaging sensor as the imaging sensor is rotated. The controller is configured to adjust a rotational orientation of the one or more images displayed by the display based upon the measured current.

The catheter system can include one or more of the following features. The imaging sensor can be an optical coherence tomography (OCT) imaging sensor. The catheter body can further include a cutter configured to rotate with the driveshaft. The imaging sensor can be attached to the cutter. The catheter system can further include a nosecone attached to the distal end of the catheter body and configured to pivot relative thereto. The sensor can be a current sense resistor. The one or more images can be displayed on the display as a sector view. The controller can be configured adjust a rotational orientation of the one or more images by delaying projection of the one or more images on the display based upon the measured current. The controller can be further configured to adjust a rotational orientation of the one or more images based upon a predetermined reference current value and gain value. The controller, drive motor, and sensor can be part of a drive assembly configured to drive the catheter.

Also described is a catheter drive assembly that includes a drive motor, a sensor, and a controller. The drive motor is configured to rotate a drive shaft and imaging sensor of a catheter at first index pulse. The sensor is configured to measure an amount of current drawn by the drive motor as the drive shaft is rotated at the first index pulse. The controller is configured to determine a second index pulse based upon the measured current and the first index pulse and send the second index pulse to a display so as to allow display of images gathered by the imaging sensor that are adjusted for rotational lag. The second index pulse is delayed relative to the first index pulse.

This and other catheter drive assemblies can include one or more of the following features. The sensor can be a current sense resistor. The controller can be configured to determine a second index pulse further based upon a predetermined reference current value and gain value. The imaging sensor can be an optical coherence tomography (OCT) imaging sensor.

Also described is a non-claimed method of correcting for rotational lag that includes: obtaining a reference current value for an imaging catheter when there is no torque on a driveshaft of the catheter; measuring an amount of current delivered to a drive motor to rotate the driveshaft during a procedure; determining that there has been rotational lag of the driveshaft by determining that the measured amount of current is above the reference current value by a determined amount; and correcting a displayed image for the rotational lag based upon the determined amount.

This and other non-claimed methods can include one or more of the following features. The measuring, determining, and correcting steps can be performed during use of the catheter in an atherectomy procedure. Correcting a displayed image for the rotational lag based upon the determined amount can include delaying an index pulse to the displayed image so as to delay projection of the image. The imaging catheter can be an optical coherence tomography (OCT) imaging catheter, and the displayed image can be an OCT image.

Also described is a non-claimed method of correcting for rotational lag that includes: obtaining a correlation between an amount of rotational distortion in an image and an amount of current drawn by a drive motor of an imaging catheter; measuring an amount of current delivered to the drive motor to rotate a driveshaft of the imaging catheter with the drive motor; when the measured current is above a predetermined value, using the correlation to determine a rotational adjustment value; and adjusting an orientation of an image based upon the rotational adjustment value.

This and other non-claimed methods can include one or more of the following features. The measuring, using, and adjusting steps can be performed during use of the catheter in an atherectomy procedure. Adjusting an orientation of an image can include delaying an index pulse to a display so as to delay projection of the image on the display. The imaging catheter can be an optical coherence tomography (OCT) imaging catheter, and the image can be an OCT image.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIGS. 1A - 1D are schematics showing an exemplary atherectomy catheter. FIG. 1A is a side view of the atherectomy catheter. FIG. 1B shows a catheter where the cutter is exposed. FIG. 1C is a second example of an atherectomy catheter. FIG. 1D shows a cross section of the catheter.
FIG. 2 is a schematic showing a dynamic adjustment to compensate for rotational lag.
FIG. 3 is a flow chart summarizing the steps in calibrating a catheter.
FIG. 4 is a flow chart summarizing the steps of correcting for rotational lag when using an atherectomy catheter.
FIG. 5A is an image from an atherectomy catheter as the cutting element is starting to open.
FIG. 5B is an image from an atherectomy catheter that is fully open where there is no dynamic image correction
FIG. 5C is an image from an atherectomy catheter that is fully open with correction.

### DETAILED DESCRIPTION

Described herein are methods and assemblies for addressing rotational lag of an imaging catheter (e.g., an atherectomy catheter) in a simple, cost effective, and dynamic way. The methods and assemblies described herein are configured to determine the existence of rotational lag at the imaging element and dynamically compensate for the lag before the image is recorded and displayed.

The catheters described herein can include an elongate flexible catheter body and a cutting element. In some embodiments, the cutting element can be an annular rotatable cutter configured to rotate to shear tissue away from the vessel wall. In other embodiments, cutting element can include a distal tip having a proximal-facing cutting edge configured to scrape tissue away from the vessel wall. The catheters described herein can further include on-board imaging, such as optical coherence tomography (OCT) imaging. The optical fiber for the OCT imaging can, for example, extend substantially along the longitudinal axis of the catheter body. In some embodiments, the optical fiber can be attached to the rotatable cutter and configured to rotate therewith. In other embodiments, the optical fiber can be attached to a separate imaging shaft.

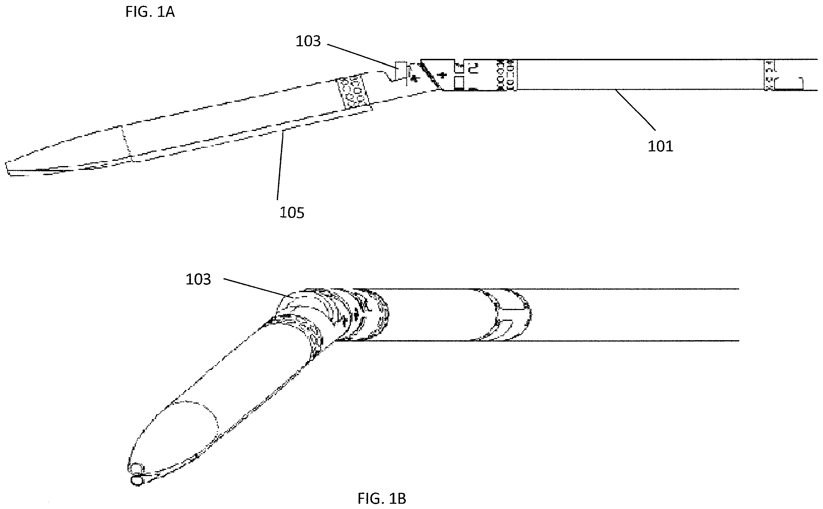

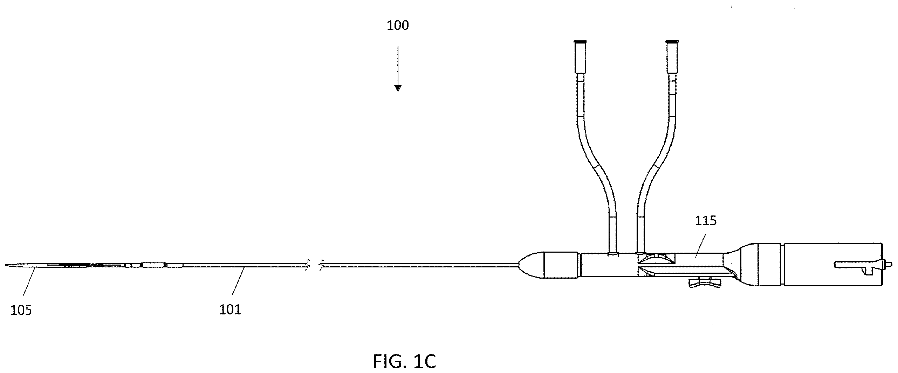

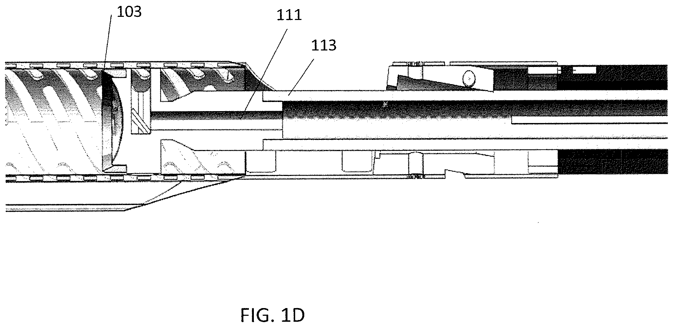

FIGS. 1A -1C show an exemplary atherectomy catheter. Referring to FIG. 1A, the atherectomy catheter 100 can include a catheter body 101, a cutter 103 at a distal end of the catheter body 101 attached to a driveshaft 1 13, and a nosecone 105 at a distal end of the catheter body 101. The nosecone 105 can further include a cutting window through which the cutting edge 112 of the cutter 103 can be exposed. The nosecone 105 can be configured to deflect away from the longitudinal axis of the catheter body at an angle. In use, this deflection can expose the cutter 103 through the cutting window and/or radially push the cutter 103 into a wall of the vessel in which the atherectomy catheter is inserted. As shown in FIG. 1D, the catheter 100 can further include an on-board imaging system, such as optical coherence tomography (OCT). The optical fiber 111 for the OCT imaging element can run through the center of the driveshaft 113. Further, the optical fiber 111 can be attached to the cutter 103 and be configured to spin therewith.

Further, the catheter 100 may include a handle 115 and/or drive system that includes controls for regulating the displacement, rotation, and/or imaging capabilities of the imaging system. The catheter 100 can be configured to be attached to a control system and/or imaging console for displaying the images gathered with the on-board in imaging system.

Further, the catheter 100 can be configured to work with a control system configured to correct for image distortion, e.g., to correct for rotational lag. Because a catheter is not an ideal torque transmitting entity, there will be some phase delay (θ) for which the distal end of the catheter does not rotate when the proximal end of the catheter is rotated. This phase delay can cause incorrect orientation of the image when the catheter is torqued and/or the direction of rotation changes, called rotational lag. A host of causes may contribute to potential rotational lag. These include torqueing of the catheter due to the tortuosity of the vasculature or force exerted by the operator, cutter position, the type of plaque being excised, and/or any other factors that may apply a torsional load on the catheter.

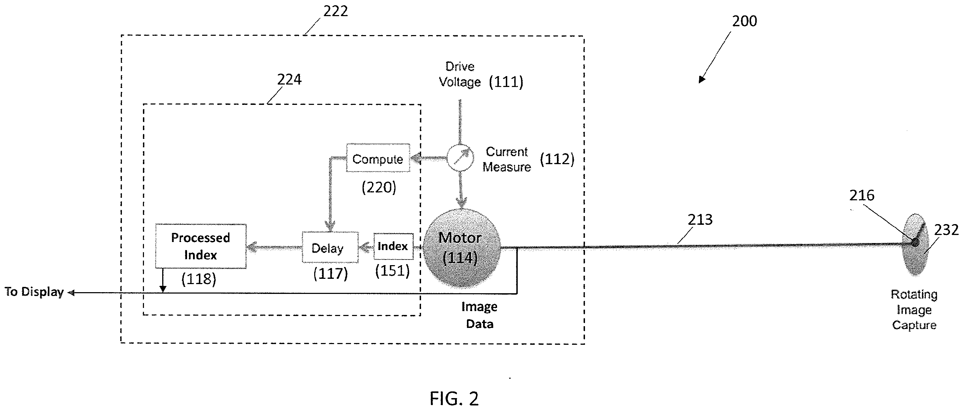

FIG. 2 shows a diagram of an atherectomy system 200 configured to correct for rotational lag. The system includes a drive assembly 222, which can be a drive assembly such as those described in U.S. Patent Application No. 14/400,151, filed November 10, 2014, titled "ATHERECTOMY CATHETER DRIVE ASSEMBLIES", now Patent No. 9,345,398 and PCT Patent Application No. PCT/US2016/041193, filed July 6, 2016, titled "SELF-ALIGNMENT MECHANISM FOR IMAGING CATHETER AND DRIVE ASSEMBLY", Publication No. WO 2017/007853. The drive assembly 222 includes a drive shaft motor 114 configured to rotate the drive shaft 213 (and the imaging sensor 216 at the distal end thereof) of the catheter. As the drive shaft motor 114 rotates, it provides an index pulse, which can be read by a controller 224 at (e.g., at 151) every time the motor drive shaft passes a set reference point (e.g., a top dead center position). The drive assembly further includes a drive voltage source 111 to power the drive shaft motor.

In use, the motor 114 can rotate the drive shaft 213 and imaging sensor 216 to gather image data from a circumferential area 232 (e.g., within a body lumen, such as a blood vessel). The image data can be sent back through a controller 224. Simultaneously, the controller 224 can determine the index pulse 151 of the motor 114. The imaging data can then be sent to the display with a set timestamp based upon the index, e.g., such that the top of the displayed OCT image corresponds to the top dead center position on the drive shaft. As torque is applied to the driveshaft 213, however, the displayed image will be rotationally misaligned (or delayed) relative to the actual position of the sensor 216 if a delay is not added to the index pulse.

As such, a current sensor 112 can be disposed between the drive voltage source 111 and the drive shaft motor 114 for continuous monitoring of the current drawn by the drive shaft motor 114. In some variations, the current is sensor can be integral with the motor 114. In other variations, the current sensor 112 can be a current sensor resistor placed in series with the drive shaft motor 114. The instantaneous current of the motor 114 will vary as a function of the torsional load, meaning that the current drawn will change as a function of the torsional strain the catheter experiences. By measuring the instantaneous current of the motor 114, the torsional load on the motor 114 may be estimated.

The controller 224 can be configured, at 115, to correlate the amount of torque load experienced at the distal end of the catheter (i.e., at the imaging sensor 116) to the change in current drawn from the drive motor 114 (i.e., to compute the rotational lag). Based upon the rotational lag, the controller 224 can implement a delay 117 in the index pulse. The delay 117 can result in a new processed index 118. This processed index 118 instructs the display to delay the presentation of the data in the OCT image by a set time in order to rotationally align the image with the original reference orientation (e.g.., place the image back in the top dead center position).

Some methods of correcting for rotational lag can include two separate steps: (1) calibrating the system to determine the additional motor current draw relative to torsional load; and (2) then determining the instantaneous motor current of the system during use and making a correction in the resulting image.

In some methods, the first step can be performed largely empirical, i.e., one or more different models of catheters can be repeatedly tested in order to obtain a correlation between the change in current and the amount of rotational distortion experienced by that particular model of catheter. In some methods, two important parameters, the offset value and the gain value, can be calculated for any given catheter model and then used to determine the correct correlation between the current and amount of rotational distortion. The offset value corresponds to an amount of current that the drive motor draws when there is no lag. The offset value is thus equivalent to the current that the drive motor draws when the catheter is in a relaxed position with the cutter closed. The gain value is a numeric value that allows correction of the rotational image based upon the difference between the measured current and the offset value.

In some methods, the second step can be performed dynamically by a controller. That is, once a particular model of catheter has been calibrated in the first step for torsional resistance as a function of rotational distortion (i.e., once the offset value and gain value have been determined for the model), it is possible to estimate the instantaneous rotational distortion for that particular catheter/catheter model and apply an appropriate amount of correction to the resulting image. Referring back to FIG. 2, doing so can include measuring the instantaneous current with sensor 112. The adjustment for rotational lag can be performed dynamically during the use of the catheter, i.e., without direction or input from the user. As a result, the user can advantageously view an image that truly corresponds to what the optical fiber is detecting at the distal end of the catheter or at the cutting region without undesired rotational distortion of the image.

### Steps for calibrating catheter

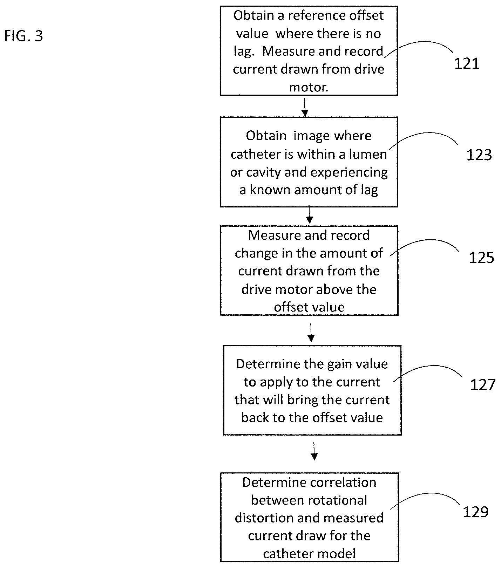

FIG. 3 is a flowchart detailing exemplary steps for calibrating a particular model of catheter, such as catheter 100, to determine the correlation between the additional motor current drawn by the motor and torsional load. Catheters considered to be a different model can be, for example, those where the driveshafts have different characteristics (e.g., dimensions, spring, etc.) such that the resulting relationship between torsion and current draw will be different. At step 121, a reference current value (called the offset value) is determined. The offset value is the current reading obtained when the catheter is in a relaxed, idle state where the catheter is outside the body cavity and where the cutter is closed. At step 123, an image can be obtained with the imaging sensor while the catheter is in the relaxed and idle state. This idle image can have a particular set orientation or rotational position (e.g., which can be determined based upon markers in the image).

At step 125, an amount of torque can be placed on the driveshaft, such as by opening the nosecone to expose the cutter. Due to the increased torque, the current drawn by the motor will be some amount over the offset. This change in current over the offset value can be measured and recorded. The gain value may then be determined, based upon equation 1 below: $R = C \left(M-O\right) G$ (Equation 1) where R is the radial adjustment value in degrees (i.e., the amount of adjustment required to place the resulting image back at the correct rotational orientation), C is a constant (in degrees/amps), O is the offset value in amps, M is the measured current in amps, and G is the gain value. That is, at this step, the appropriate gain value for the catheter model can be determined so as to rotate the image back to it's original idle position. As shown in Table 1 (which shows experimentally gathered values of offset, gain, and R), the gain value should be the same for a particular catheter model regardless of the value of R. This step can be performed, for example, empirically.

**Table 1: Experimental Results of Single Model of Catheter**

| **Catheter Number** | **Offset** | **Gain** | **Clocking degrees (R)** |
|---|---|---|---|
| 1 | 400 | 25 | 90 |
| 2 | 400 | 25 | 95 |
| 27 | 400 | 25 | 170 |
| 20 | 400 | 25 | 120 |
| 35 | 400 | 25 | 30 |
| 13 | 400 | 25 | 100 |
| 21 | 400 | 25 | 160 |
| 10 | 400 | 25 | 120 |
| 7 | 400 | 25 | 90 |
| 4 | 400 | 25 | 135 |
| 11 | 400 | 25 | 100 |
| 26 | 400 | 25 | 170 |
| 19 | 400 | 25 | 100 |
| 23 | 400 | 25 | 90 |
| 16 | 400 | 25 | 80 |
| 12 | 400 | 25 | 85 |
| 28 | 400 | 25 | 90 |
| 5 | 400 | 25 | 170 |
| 24 | 400 | 25 | 100 |
| 17 | 400 | 25 | 80 |

In some examples, Equation 1 can be considered valid for M greater than or equal to O. If M is less than O, then R (or the radial adjustment) can be considered zero.

Thus, at the calibration step, the offset value and gain values are determined so as to rotate an image back to its original position (i.e., with a known or observed R). At step 129, then, the correlation for the particular catheter can be determined (i.e., C, G, and O can be filled into Equation 1, allowing the rotational adjustment to be determined going forward depending upon the measured current (M).

### Using a catheter calibrated for rotational lag

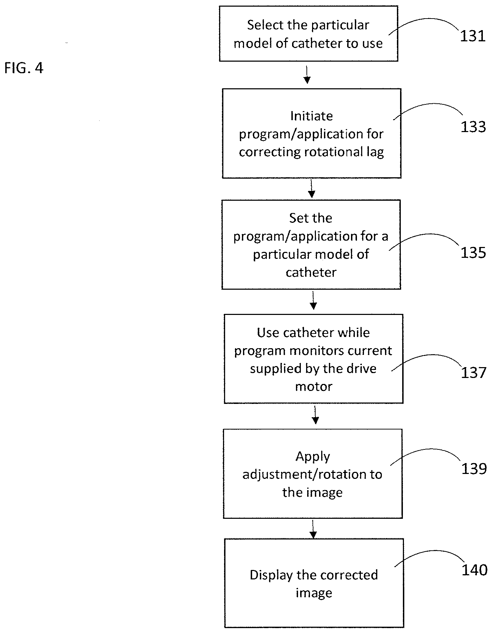

FIG. 4 is a flow chart showing the steps for dynamically correcting for rotational lag using a calibrated catheter. At step 131, an operator select a particular catheter. At step 133, the operator initiates the program for dynamically and instantaneously correcting for rotational lag. The program can be based upon the determined correlation for the catheter model from step 129 of FIG. 3. As such, the appropriate correlation can be selected at step 135 (e.g., by entering the particular catheter model identification number in the controller). At step 137, the user can operate the catheter (e.g., as part of an atherectomy procedure) while the program monitors the current supplied to the drive motor against the offset current value. At step 139, if the measured current goes above the offset value, then the value R can be obtained (based upon equation 1 and the measured current). Based upon the value R, an amount of adjustment or rotation can be applied to the image to ensure that the image maintains the correct orientation. Once adjusted, the image will be captured and displayed for the user (step 140).

There are a number of ways that the rotational lag may be compensated/adjusted for using the determined radial adjustment value (R). In one example, the image displayed can be delayed. This is based on the observation that the imaging system (e.g., OCT) provides a continuous stream of imaging data as the catheter is in operation. As the imaging element sweeps along with the drive motor, an image is swept out radially. The image displayed shown is the data obtained for that image and as it relates to a reference (e.g., the top-dead center). When rotational lag is present and twists the optical element, there is a misalignment between the top-dead center as it is defined the drive assembly and the image taken at the end of the imaging element. Because there is a delay in the detecting the true image at the end of the imaging element, by intentionally delaying the top-dead center pulse that is sent along with the image date for a set time (based on Equation 1), the image displayed can be aligned with the image detected. In such an example, the time delay may be equated to the radial adjustment value (R) according to Equation 2: $T = \left(R\times 60\right) / \left(RPM\times 360\right)$ (Equation 2) where T is the time in seconds, R is the radial adjustment value, and RPM is the speed of the drive motor in revolutions per minute. Accordingly, the index pulse can be delayed and sent from the drive assembly to the display at the delayed pulse so as to maintain the orientation of the image (i.e., the top dead center position). In some embodiments, the image can be adjusted once per revolution of the driveshaft (i.e., once per input pulse) using the controller in the drive assembly.

The methods and systems for dynamically correcting rotational lag may also include associated software programs, and applications that are able to correlate the amount of change in current from the drive motor or other electrical property and an amount of rotational lag.

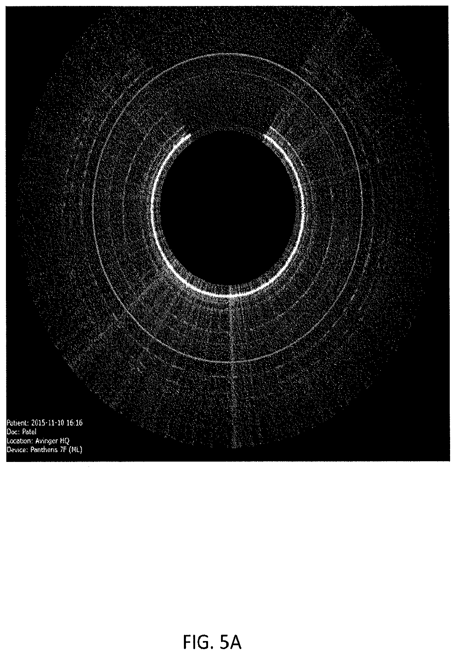

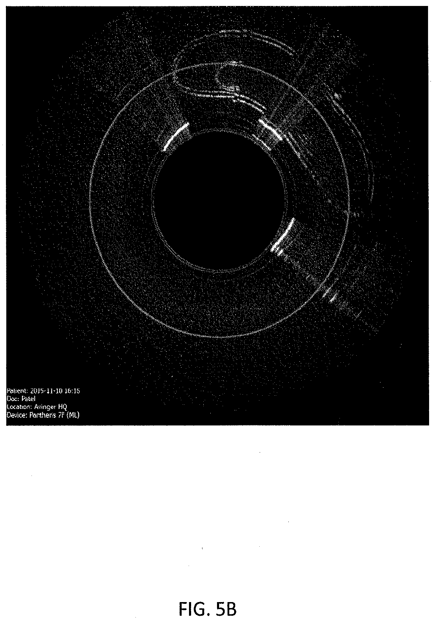

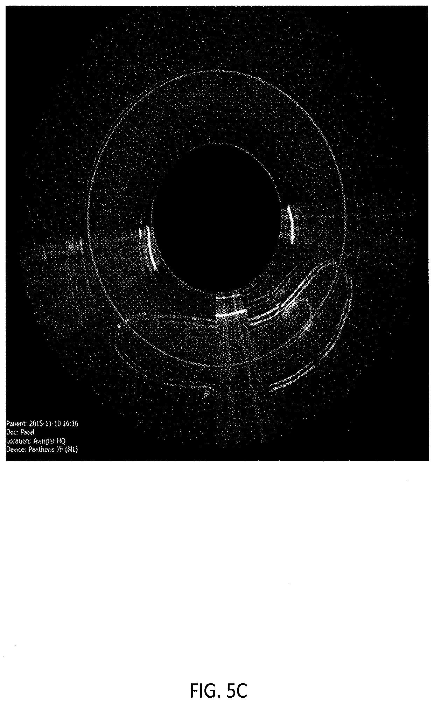

FIGS. 5A-C show the dynamic rotational distortion correction. FIG. 5A shows an OCT image of a vessel as the catheter cutter is being exposed. The cutter is shown at the 12 o'clock position (the inverted horseshoe shaped bright line corresponds to the housing surrounding the cutter while the opening corresponds to the cutter and the area of the vessel imaged). FIGS. 5B and 5C show images taken by the imaging element with correction (FIG. 5C) and without correction (FIG. 5B) when the cutter is fully opened. Without correction, as shown in FIG. 5B, the image has been rotated counter-clockwise about 130 degrees such that the exposed cutter is in the 8 o'clock position. This rotated orientation (caused by rotational lag) can be confusing to the user, given that the actual orientation of the cutter within the vessel has not changed. In contrast, with correction, as shown in FIG. 5C, the cutter has not been rotated relative to its position in FIG. 5A and is still positioned approximately at the 12 o'clock position. Ultimately, being able to see an image of the body lumen with a true orientation will provide the user an intuitive and more efficient way for either imaging or ablative procedures.

Although the controller configured to correct for rotational distortion is described herein as being located within the drive assembly, it is to be appreciated that it could be positioned elsewhere. For example, in one embodiment, a controller on the display can continuously monitor the current and update the sector view accordingly. That is, rather than assuming that every rotation occurs at a constant rotation, the controller could map current draw across each rotation and adjust the pulse gaps according to the current draw.

The methods and systems described herein provide a simple way for correcting rotational lag within an atherectomy catheter or any catheter that includes an imaging system. The rotational lag correction provided for here advantageously corrects the signal prior to an image being displayed. A correction at this stage requires less steps and less complex software programs for correcting the image. Furthermore, the systems and methods described herein provide for continuous monitoring and correction for rotational lag experienced such that the image seen always corresponds to what the imaging fiber is seeing without the need for operator intervention.

In some examples, an analog-to-digital converter may be used to process the information received and transmit the signal information to a processor or microprocessor. Further, signal processing may also include requisite low pass filters as needed. The current changes are indexed and processed to determine a relation between the changes in current and the amount of rotational distortion experienced. Finally, while the present system utilizes changes in current to correlate to an amount of rotational lag experienced, it is also conceivable to use other electrical properties of the system such as changes in voltage, resistance, impedance and so forth, for achieving similar measurements.

The methods and assemblies for correcting rotational lag can be used with a variety of catheters and/or motor assemblies. For example, the methods and assemblies can be used with the atherectomy catheters described in U.S. Patent Application No. 15/072,272, filed March 16, 2016, titled "ATHERECTOMY CATHETERS DEVICES HAVING MULTI-CHANNEL BUSHINGS", Publication No. US-2016-0192962-A1 and U.S. Patent Application No. 15/076,568, filed March 31, 2016, titled "ATHERECTOMY CATHETERS AND OCCLUSION CROSSING DEVICES", now Patent No. 9,498,247.

Although described herein as being used with an OCT system, it should be understood that the systems and methods of correcting rotational lag can be used with other types of rotational imaging, such as intravascular ultrasound (IVUS).

In the systems and methods described, there may be programs that are able to continuously, dynamically, and instantaneously correct for rotational lag based upon the current sensed. In some embodiments, the programs are part of a display assembly and/or a drive assembly. In other embodiments, the programs may be controlled via various telecommunication devices such as laptops, desktops, mobile devices, tablets, and so forth.

## Claims

1. A catheter system (100, 200), comprising:
a catheter body (101) having a drive shaft (113, 213);
an imaging sensor (216) fixed relative to the distal end of the driveshaft and configured to rotate therewith;
a drive motor (114) configured to rotate the drive shaft;
a sensor (112) configured to measure an amount of current drawn by the drive motor as the drive shaft is rotated;
a display configured to display one or more images obtained by the imaging sensor as the imaging sensor is rotated; and
a controller (224) configured to adjust a rotational orientation of the one or more images displayed by the display based upon the measured current.

2. The catheter system of claim 1, wherein the sensor (112) is a current sense resistor.

3. The catheter system of claim 1 or 2, wherein the imaging sensor (216) is an optical coherence tomography (OCT) imaging sensor.

4. The catheter system of any one of the preceding claims, wherein the catheter body (101) further includes a cutter (103) configured to rotate with the driveshaft (113, 213).

5. The catheter system of claim 4, wherein the imaging sensor (216) is attached to the cutter (103).

6. The catheter system of any one of the preceding claims, further comprising a nosecone (105) attached to the distal end of the catheter body (101) and configured to pivot relative thereto.

7. The catheter system of any one of the preceding claims, wherein the one or more images are displayed on the display as a sector view.

8. The catheter system of any one of the preceding claims, wherein the controller (224) is configured to adjust a rotational orientation of the one or more images by delaying projection of the one or more images on the display based upon the measured current.

9. The catheter system of any one of the preceding claims, wherein the controller (224) is further configured to adjust a rotational orientation of the one or more images based upon a predetermined reference current value and gain value.

10. The catheter system of any one of the preceding claims, wherein the controller (224), drive motor (114), and sensor (112) are part of a drive assembly (222) configured to drive the catheter.

## Patentansprüche

1. Kathetersystem (100, 200), das Folgendes umfasst:
einen Katheterkörper (101), der einen Antriebsschaft (113, 213) aufweist;
einen Bildsensor (216), der relativ zu dem distalen Ende des Antriebsschafts befestigt und konfiguriert ist, um sich damit zu drehen;
einen Antriebsmotor (114), der konfiguriert ist, um den Antriebsschaft zu drehen;
einen Sensor (112), der konfiguriert ist, um eine Strommenge zu messen, die durch den Antriebsmotor verbraucht wird, während der Antriebsschaft gedreht wird;
eine Anzeige, die konfiguriert ist, um ein oder mehrere Bilder anzuzeigen, die durch den Bildsensor erhalten werden, während der Bildsensor gedreht wird; und
eine Steuerung (224), die konfiguriert ist, um eine Drehausrichtung des einen oder der mehreren Bilder, die durch die Anzeige angezeigt werden, basierend auf dem gemessenen Strom anzupassen.

2. Kathetersystem nach Anspruch 1, wobei der Sensor (112) ein Stromerfassungswiderstand ist.

3. Kathetersystem nach Anspruch 1 oder 2, wobei der Bildsensor (216) ein Bildsensor einer optischen Kohärenztomographie (*optical coherence tomography* - OCT) ist.

4. Kathetersystem nach einem der vorhergehenden Ansprüche, wobei der Katheterkörper (101) ferner eine Schneidvorrichtung (103) beinhaltet, die konfiguriert ist, um sich mit dem Antriebsschaft (113, 213) zu drehen.

5. Kathetersystem nach Anspruch 4, wobei der Bildsensor (216) an der Schneidvorrichtung (103) angebracht ist.

6. Kathetersystem nach einem der vorhergehenden Ansprüche, das ferner einen Nasenkonus (105) umfasst, der an dem distalen Ende des Katheterkörpers (101) angebracht ist und konfiguriert ist, um relativ dazu zu schwenken.

7. Kathetersystem nach einem der vorhergehenden Ansprüche, wobei das eine oder die mehreren Bilder auf der Anzeige als Sektoransicht angezeigt werden.

8. Kathetersystem nach einem der vorhergehenden Ansprüche, wobei die Steuerung (224) konfiguriert ist, um eine Drehausrichtung des einen oder der mehreren Bilder durch Verzögern einer Projektion des einen oder der mehreren Bilder auf der Anzeige basierend auf dem gemessenen Strom anzupassen.

9. Kathetersystem nach einem der vorhergehenden Ansprüche, wobei die Steuerung (224) ferner konfiguriert ist, um eine Drehausrichtung des einen oder der mehreren Bilder basierend auf einem zuvor bestimmten Referenzstromwert und einem Verstärkungswert anzupassen.

10. Kathetersystem nach einem der vorhergehenden Ansprüche, wobei die Steuerung (224), der Antriebsmotor (114) und der Sensor (112) Teil einer Antriebsanordnung (222) sind, die konfiguriert sind, um den Katheter anzutreiben.

## Revendications

1. Système de cathéter (100, 200), comprenant :
un corps de cathéter (101) ayant un arbre d'entraînement (113, 213) ;
un capteur d'imagerie (216) fixe par rapport à l'extrémité distale de l'arbre d'entraînement et configuré pour tourner avec celle-ci ;
un moteur d'entraînement (114) conçu pour faire tourner l'arbre d'entraînement ;
un capteur (112) configuré pour mesurer une quantité de courant consommée par le moteur d'entraînement à mesure que l'arbre d'entraînement tourne ;
un dispositif d'affichage configuré pour afficher une ou plusieurs images obtenues par le capteur d'imagerie à mesure que le capteur d'imagerie tourne ; et
un dispositif de commande (224) configuré pour régler une orientation de rotation de la ou des images affichées par le dispositif d'affichage en fonction du courant mesuré.

2. Système de cathéter selon la revendication 1, dans lequel le capteur (112) est une résistance de détection de courant.

3. Système de cathéter selon la revendication 1 ou 2, dans lequel le capteur d'imagerie (216) est un capteur d'imagerie de tomographie par cohérence optique (TCO).

4. Système de cathéter selon l'une quelconque des revendications précédentes, dans lequel le corps de cathéter (101) comporte en outre un instrument tranchant (103) conçu pour tourner avec l'arbre d'entraînement (113, 213).

5. Système de cathéter selon la revendication 4, dans lequel le capteur d'imagerie (216) est attaché à l'instrument tranchant (103).

6. Système de cathéter selon l'une quelconque des revendications précédentes, comprenant en outre un cône avant (105) attaché à l'extrémité distale du corps de cathéter (101) et conçu pour pivoter par rapport à celle-ci.

7. Système de cathéter selon l'une quelconque des revendications précédentes, dans lequel la ou les images sont affichées sur le dispositif d'affichage sous forme de vue sectorielle.

8. Système de cathéter selon l'une quelconque des revendications précédentes, dans lequel le dispositif de commande (224) est configuré pour régler une orientation de rotation de la ou des images en retardant la projection de la ou des images sur le dispositif d'affichage en fonction du courant mesuré.

9. Système de cathéter selon l'une quelconque des revendications précédentes, dans lequel le dispositif de commande (224) est en outre configuré pour régler une orientation de rotation de la ou des images en fonction d'une valeur de courant de référence et d'une valeur de gain prédéterminées.

10. Système de cathéter selon l'une quelconque des revendications précédentes, dans lequel le dispositif de commande (224), le moteur d'entraînement (114) et le capteur (112) font partie d'un ensemble d'entraînement (222) configuré pour entraîner le cathéter.
